# FASCICULE DE BREVET EUROPEEN

(11) **EP 2 928 895 B1**
(45) Date de publication et mention de la délivrance du brevet: **08.01.2020**
(21) Numéro de dépôt: 13815073.5
(22) Date de dépôt: 06.12.2013
(51) Int. Cl.: C07D 493/04, C08G 63/68

(54) **COMPOSES A BASE DE DIANHYDROHEXITOL ET COMPOSITIONS RETICULABLES LES COMPRENANT**
DIANHYDROHEXIT-DERIVATE UND DEREN VERWENDUNG IN VERNETZBAREN MASSEN
DERIVATIVES OF DIANHYDROHEXITOL AND THEIR USE AS CROSSLINKABLE COMPOSITIONS

(30) Priorité: 06.12.2012 FR 1261725
(43) Date de publication de la demande: 14.10.2015
(73) Titulaire: Roquette Frères, 62136 Lestrem (FR); Centre National de la Recherche Scientifique (C.N.R.S.), 75794 Paris Cedex 16 (FR); UNIVERSITE DE MONTPELLIER, 34090 Montpellier (FR); Ecole Nationale Superieure De Chimie Montpellier (ENSC), 34000 Montpellier (FR)
(72) Inventeur: IBERT, Mathias, F-59930 La Chapelle D'armentieres (FR); BUFFE, Clothilde, F-59160 Lomme (FR); SAINT-LOUP, René, F-59160 Lomme (FR); FERTIER, Laurent, F-86000 Poitiers (FR); ROBIN, Jean-Jacques, F-34830 Clapiers (FR); GIANI, Olivia, F-34920 Le Crès (FR); JOLY-DUHAMEL, Christine, F-34150 Gignac (FR)
(74) Mandataire: Plasseraud IP
(86) Numéro de dépôt international: PCT/FR2013/052974
(87) Numéro de publication internationale: WO 2014/087113

(56) Documents cités:
- WO-A1-2008/031592
- WO-A1-2011/048739
- WO-A2-2013/066461
- DATABASE CAPLUS [Online] CHEMICAL ABSTRACTS SERVICE, COLUMBUS, OHIO, US; NAKAJIMA, YUKA ET AL: "Electrophotographic photoconductor showing improved durability and electric characteristics, process cartridge, and electrophotographic apparatus", XP002694182, extrait de STN Database accession no. 2003:116666 -& JP 2003 043711 A (CANON INC., JAPAN) 14 février 2003 (2003-02-14)
- PHILIP, BIJU ET AL: "Optically active poly(ester-amide)s: synthesis and characterization", POLYMER INTERNATIONAL , 50(12), 1318-1323 CODEN: PLYIEI; ISSN: 0959-8103, vol. 50, 2001, pages 1318-1323, XP002694183,
- Lidia Jasinska ET AL: "Unsaturated, biobased polyesters and their cross-linking via radical copolymerization", Journal of Polymer Science Part A: Polymer Chemistry, vol. 48, no. 13, 1 July 2010 (2010-07-01), pages 2885-2895, XP055062218, ISSN: 0887-624X, DOI: 10.1002/pola.24067

## Description

### DOMAINE TECHNIQUE DE L'INVENTION

La présente invention concerne de nouveaux monomères, des compositions réticulables les comprenant, ces compositions permettant notamment de former des revêtements sur différents types de substrats. La présente invention concerne en particulier des compositions comprenant des monomères à base de dianhydrohexitol, leurs utilisations et les revêtements polymères obtenus à partir de ces compositions.

### ART ANTERIEUR

De nombreuses industries ont besoin de compositions permettant de former des revêtements sur des substrats. Il peut s'agir par exemple de revêtements protecteurs, décoratifs, ou de traitement de surface. Il existe déjà un grand nombre de compositions réticulables pour cet usage dans la littérature et dans le commerce. Ces compositions consistent pour la plupart en un mélange de monomères polymérisables fabriqués par l'industrie chimique à partir de dérivés du pétrole.

Cependant, dans le contexte actuel de la diminution progressive des ressources en produits pétroliers, il est de plus en plus intéressant de remplacer les produits d'origine pétrolière par des produits d'origine naturelle. Il est donc nécessaire de trouver de nouveaux composés utiles à la fabrication de polymères.

L'utilisation de polymères biosourcés, c'est-à-dire réalisés à partir de matières premières d'origine naturelle, a déjà été décrite. En particulier, la publication de L. Jasinska et de C. E. Koning (« Unsaturated, biobased polyesters and their cross-linking via radical copolymerization », Journal of Polymer Science Part A: Polymer Chemistry, Volume 48, Issue 13, pages 2885-2895, 1 Juillet 2010) décrit la préparation de polyesters insaturés ayant des températures de transition vitreuse (Tg) relativement élevées, i.e. supérieures à 45°C, pour des applications comme revêtement. Ces polyesters insaturés sont obtenus par polymérisation d'isosorbide avec de l'anhydride maléique et optionnellement de l'acide succinique. Pour pouvoir être utilisés en tant que revêtement, ces polyesters insaturés doivent cependant être réticulés. Les polymères sont donc mis en solution ou en suspension avec un agent réticulant, puis appliqués sur le substrat, et enfin réticulés après évaporation du solvant. Le fait que la réalisation du revêtement nécessite plusieurs étapes successives est contraignant.

Des composés polyesters saturés, notamment des polyesters à base d'acide succinique et d'isoidide, sont également décrits dans la demande WO 2008/031592. Ces composés sont solides, ce qui leur permet d'être utilisés dans des compositions de revêtement poudre dites « powder coating ». Un premier inconvénient est que ces composés ne sont pas liquides, ce qui peut poser des problèmes de manipulation et nécessite d'utiliser des techniques très particulières d'application de ces compositions de « powder coating ». Par ailleurs, comme il apparaît dans les exemples, il est également nécessaire d'utiliser ces composés avec un agent réticulant, et non avec un amorceur, tels que les polyisocyanates pour former le revêtement. Or, ces composés doivent être manipulés avec beaucoup de précaution car ils sont irritants voire toxiques pour certains. La Demanderesse a pu également constater que, notamment dans les conditions des exemples de la présente demande, le di(succinate d'éthyle) isosorbide n'est pas réactif, et ceci même en présence d'amorceur.

Un des objectifs de la présente invention est de proposer une composition réticulable permettant de fabriquer des revêtements de manière plus facile, de préférence sans étape de séchage.

La demande de brevet WO 2011/048739 décrit des compositions comprenant des composés (méth)acrylates cycliques ayant une formule générale spécifique, lesdits composés pouvant être obtenus à partir d'isosorbide. Ces composés constituent des monomères susceptibles de polymériser entre eux par voie radicalaire. Un des problèmes rencontrés lors de l'utilisation de telles compositions est que ces compositions sont fortement instables. C'est pourquoi il est nécessaire de stocker ces compositions dans un lieu à l'abri de la lumière et à basse température, ou bien de leur ajouter un agent inhibiteur de polymérisation radicalaire comme l'hydroquinone par exemple, empêchant la polymérisation des composés (méth)acrylates cycliques entre eux.

La demande JP2003043711 décrit quant à elle un photoconducteur comprenant un pigment phtalocyanine particulier, ce photoconducteur comprenant un revêtement à base d'un polyester et de microparticules de taille définie. Le polyester peut contenir des motifs 1,2-cyclohexane diméthanol. Par ailleurs, contrairement aux matériaux obtenus à partir des composés selon l'invention, la Demanderesse a pu constater que le matériau obtenu à partir de di(maléate d'éthyle) de 1,2-cyclohexanediméthanol présente une tenue thermique peu satisfaisante.

Il serait avantageux par conséquent de disposer de nouveaux monomères, pouvant entrer comme constituant de compositions réticulables présentant une meilleure stabilité que les compositions déjà connues et permettant également d'obtenir des revêtements de qualité au moins équivalente, voire supérieure, par rapport aux revêtements obtenus avec les compositions déjà connues. Une meilleure conservation de la composition réticulable dans le temps, avec des conditions de stockage plus simples représente en effet un avantage important pour l'utilisateur.

C'est pour répondre à cette attente que les inventeurs ont recherché de nouvelles compositions réticulables. Dans ce contexte, ils ont trouvé de nouveaux composés à base de dianhydrohexitol utiles comme monomères. Ils ont également découvert que l'association de ce monomère avec un autre ou plusieurs autres monomères possédant des fonctions insaturées réactives permettait de fabriquer des revêtements de manière simple et efficace, pouvant avantageusement remplacer les revêtements obtenus à partir de dérivés pétrochimiques, ne présentant pas les inconvénients de l'art antérieur.

### RESUME DE L'INVENTION

L'invention a donc pour objet un composé (A) dérivé de 1,4:3,6-dianhydrohexitol porteur de deux fonctions maléate et/ou fumarate répondant aux formules : 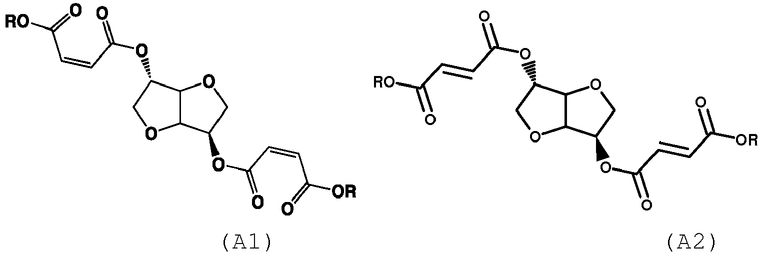 dans laquelle :
- R représente, de façon indépendante un groupe alkyle, de préférence un groupe alkyle comprenant de 1 à 10 atomes de carbone ;

Ce composé nouveau est un monomère particulièrement utile pour la fabrication de polymères, notamment de polymères réticulés.

L'invention a pour objet le polymère obtenu à partir du composé (A).

En particulier, ce monomère (A) est apte à réagir avec un monomère porteur de deux fonctions éther vinylique et/ou allylique, suivant une réaction de type accepteur-donneur.

L'invention a également pour objet une composition comprenant :
- le composé ou monomère (A) ;
- et un monomère (B) porteur de deux fonctions éther vinylique et/ou allylique.

Un objet plus particulier de l'invention porte sur un procédé de fabrication d'un revêtement polymère sur un substrat, ainsi que le revêtement susceptible d'être obtenu par ledit procédé.

### DESCRIPTION DETAILLEE

Les 1,4:3,6-dianhydrohexitols, également appelés dianhydrohexitols ou isohexides, sont des produits bien connus, obtenus par déshydratation des hexitols, c'est à dire par déshydration des sucres hydrogénés en C6 tels que le sorbitol, le mannitol et l'iditol. Les 1,4:3,6-dianhydrohexitols existent essentiellement sous la forme de trois isomères, l'isosorbide, l'isomannide et l'isoidide, selon la configuration des deux fonctions hydroxy.

Dans la présente demande, le terme « 1,4:3,6-dianhydrohexitols » désigne l'isosorbide, l'isomannide, l'isoidide et les mélanges d'au moins deux de ces produits. Toutefois, dans la présente invention, les 1,4:3,6-dianhydrohexitols peuvent avantageusement être l'isosorbide.

Le composé ou monomère (A) selon l'invention répond aux formules chimiques suivantes : 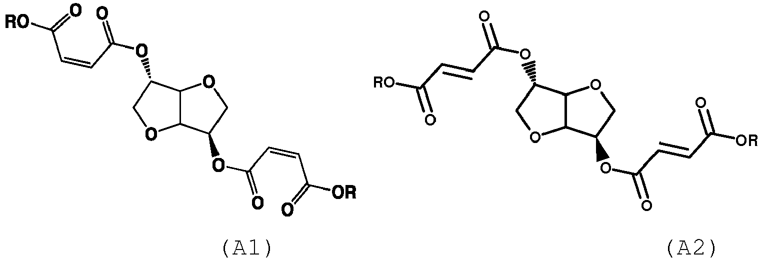 dans R représente de façon indépendante un groupe alkyle, de préférence un groupe alkyle comprenant de 1 à 10 atomes de carbone.

Ainsi, selon la configuration dela double liaison du 1,4:3,6-dianhydrohexitol, ce monomère comprend des fonctions esters fumariques et/ou esters maléiques.

Le monomère (A) est, de préférence, le dimaléate d'isosorbide(Al), variante où R est un atome d'hydrogène, le di(maléate d'alkyle)isosorbide (A1), variante où R est un groupe alkyle, le difumarate d'isosorbide (A2), variante où R est un atome d'hydrogène), le di(fumarate d'alkyle)isosorbide (A2), variante où R est un groupe alkyle), ou un mélange de ces composés. Ces composés peuvent être représentés par les formules ci-dessous : 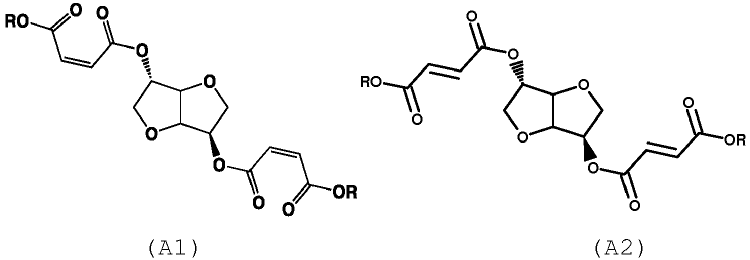 dans lesquelles R représente, de façon indépendante, un atome d'hydrogène ou un groupe alkyle, de préférence un groupe alkyle comprenant de 1 à 10 atomes de carbone.

Le composé (A) peut être obtenu à partir du 1,4:3,6-dianhydrohexitol, disponible dans le commerce. L'utilisation de 1,4:3,6-dianhydrohexitol comme matière de base pour la préparation du monomère (A) de la présente invention est particulièrement avantageuse car les 1,4:3,6-dianhydrohexitols sont biosourcés.

Le monomère (A) est un monomère susceptible d'être obtenu à partir d'acide butènedioïque et de 1,4:3,6-dianhydrohexitol par estérification ou transestérification.

L'acide butènedioïque est choisi parmi l'acide fumarique, connu également sous la dénomination d'acide E-butènedioïque, et l'acide maléique, également appelél'acide Z-butènedioïque.

Le monomère (A) est également susceptible d'être obtenu à partir d'anhydride maléique.

Différentes voies de synthèse du monomère (A) sont présentées ci-dessous. Pour des raisons de simplicité, les schémas réactionnels associés représentent les différentes voies de synthèse d'un monomère (A).

Selon une première variante, le monomère (A) est obtenu par une réaction d'estérification en faisant réagir un acide butènedioïque ou de l'anhydride maléique avec du 1,4:3,6-dianhydrohexitol selon le schéma réactionnel suivant.

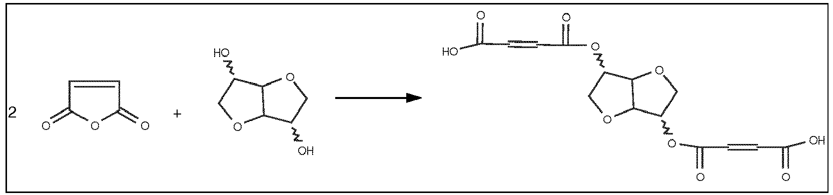

Dans ce cas, le groupe R du monomère (A) formé est un atome d'hydrogène.

La proportion molaire acide butènedioïque : 1,4:3,6-dianhydrohexitol ou anhydride maléique : 1,4:3,6-dianhydrohexitol va avantageusement de 1,5 : 1 à 100 : 1, de préférence de 2 : 1 à 5 : 1, par exemple est proche de 3 : 1.

Selon une deuxième variante, le monomère (A) est obtenu par une réaction de transestérification en faisant réagir du diester d'acide butènedioïque avec du 1,4:3,6-dianhydrohexitol selon le schéma réactionnel suivant :

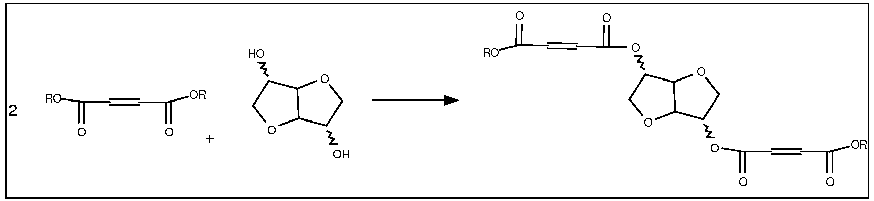

Dans ce cas, le groupe R du monomère (A) formé est un groupe alkyle.

La proportion molaire diester : 1,4:3,6-dianhydrohexitol va avantageusement de 1,5 : 1 à 100 : 1, de préférence de 2 :1 à 5 : 1, par exemple est proche de 3 : 1.

Selon une troisième variante, le monomère (A) est obtenu par une réaction d'estérification comprenant une étape de réaction d'un hémi-ester d'acide butènedioïque avec du 1,4:3,6-dianhydrohexitol selon le schéma réactionnel suivant :

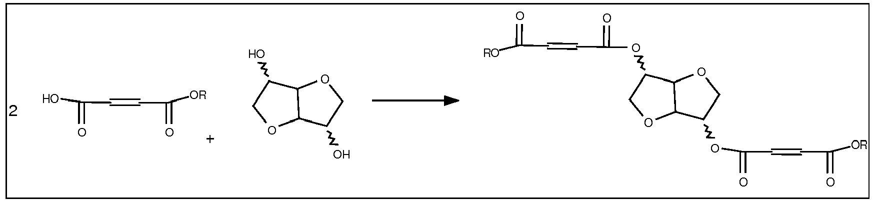

Dans ce cas, le groupe R du monomère (A) formé est un groupe alkyle.

La proportion molaire hémi-ester : 1,4:3,6-dianhydrohexitol va avantageusement de 1,5 : 1 à 100 : 1, de préférence de 2 : 1 à 5 : 1, par exemple est proche de 3 : 1.

Cette troisième variante permet d'obtenir très aisément le monomère (A), et ceci, avec un excellent rendement. Elle constitue, par conséquent, une variante toute préférée.

La description du procédé de fabrication de cette variante toute préférée est détaillée ci-après.

On peut préparer l'hémi-ester par réaction d'acide butènedioïque ou d'anhydride maléique avec un monoalcool, notamment un monoalcool primaire ou secondaire. Ce monoalcool peut comprendre de 1 à 10 atomes de carbone, de préférence de 1 à 6. Ce monoalcool est, de préférence, saturé. Il peut être choisi parmi le méthanol, l'éthanol, les isomères du propanol, du butanol, du pentanol, de l'hexanol, de l'heptanol, de l'octanol, du nonanol et du décanol. L'alcool est de préférence un alcool primaire linéaire.

On peut former cet hémi-ester en faisant réagir en excès le monoalcool avec un acide butènedioïque ou l'anhydride maléique, par exemple en utilisant le monoalcool comme solvant de la réaction.

Cette réaction peut être conduite à température ambiante, sous pression réduite. Le réacteur est généralement équipé d'un moyen d'agitation.

L'étape d'estérification peut quant à elle être réalisée dans un réacteur, dont la pression à l'intérieur de celui-ci va de 0,5 à 1,1 bar, de préférence est à pression atmosphérique. Afin de conduire la réaction d'estérification, le réacteur est équipé d'un moyen d'élimination de l'eau produite lors de la réaction afin de déplacer l'équilibre de la réaction vers le monomère (A). Il peut s'agir par exemple d'un appareil Dean Stark surmonté d'un réfrigérant. Le réacteur est généralement équipé d'un moyen d'agitation.

La réaction d'estérification peut se faire en solvant, notamment dans du toluène ou du xylène. La température dans le milieu réactionnel est de préférence comprise entre 110°C et 130°C. De préférence, la réaction est réalisée en présence de catalyseur acide, par exemple d'acide para-toluène sulfonique. Les quantités en catalyseur pourront être adaptées par l'homme du métier. A titre d'exemple, pour l'acide para toluène sulfonique, les quantités en catalyseur peuvent aller de 0,1 à 2 moles de catalyseur par mole de dianhydrohexitol.

Dans le cas où la réaction est réalisée en solvant, le procédé de fabrication du monomère (A) comprend classiquement une étape ultérieure d'évaporation du solvant, par exemple sous pression réduite. On obtient à l'issue de cette étape un brut réactionnel comprenant le monomère (A).

Le procédé de fabrication du monomère (A) peut comprendre également une étape de lavage du brut réactionnel formé lors de l'étape d'évaporation. Cette étape de lavage peut se faire à l'aide d'une solution aqueuse d'hydrogénocarbonate de sodium et/ou d'une solution de chlorure de sodium. Le monomère (A) se retrouve dans la phase organique. On peut éventuellement diluer le brut réactionnel dans de faibles quantités de solvant, par exemple de dichlorométhane, afin de faciliter l'étape de lavage.

A la suite de l'étape de lavage, on peut réaliser une étape de séchage de la phase organique, par exemple à l'aide de sulfate de magnésium anhydre.

A l'issue du procédé, on peut obtenir un monomère ou un mélange de monomères (A).

Dans le cas où l'on obtient un mélange de monomères (A), on peut réaliser une séparation de ces différents monomères, par exemple par distillation, avant qu'ils soient utilisés comme expliqué dans la suite de la description.

Il est également possible d'utiliser directement le mélange de monomères (A) sans étape de séparation.

Comme indiqué dans les exemples, le monomère (A) prend généralement la forme, à température ambiante (25°C), d'un liquide, ce liquide présentant un aspect huileux. Ceci est particulièrement intéressant car il peut donc être aisément stocké et manipulé avant utilisation. Il est également particulièrement facile de formuler des compositions à partir de ce composé.

Un autre objet de l'invention est le polymère obtenu à partir du composé (A). Ce polymère est ou non réticulé.

L'invention porte également sur une composition comprenant le composé ou monomère (A) dérivé de 1,4:3,6-dianhydrohexitol susmentionné.

La composition selon l'invention peut comprendre outre le monomère (A), un monomère (B) porteur de deux fonctions éther vinylique et/ou allylique. De préférence, la composition selon l'invention comprend outre le monomère (A), le monomère (B).

Selon une variante, le monomère (B) est un monomère de 1,4:3,6-dianhydrohexitol (B1).

Le monomère (B1) est choisi, de préférence, parmi le divinyl éther d'isosorbide (B1a), le diallyl éther d'isosorbide (B1b) ou un mélange de divinyl éther d'isosorbide (B1a) et de diallyl éther d'isosorbide (B1b). Ces composés peuvent être représentés comme suit :

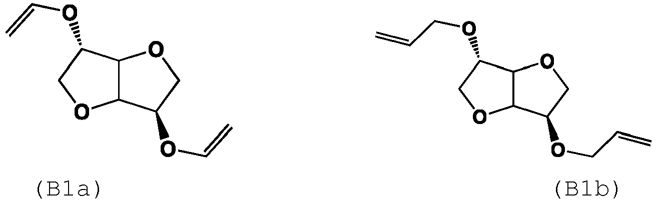

Dans le cas où le monomère (B) est un dérivé de 1,4:3,6-dianhydrohexitol, l'introduction de fonctions éther vinylique et/ou allylique sur le 1,4:3,6-dianhydrohexitol peut être réalisée selon le protocole décrit dans Zhurnal Obshchei Khimii, 1972, 42(10), p 2302-2303 par addition d'acétylène pour le dérivé vinylique ou pour le dérivé allylique via une synthèse type Williamson avec le bromure d'allyle.

Selon une autre variante, la composition peut comprendre outre le monomère (A), un monomère (B) porteur de deux fonctions éther vinylique et/ou allylique, autre que le monomère (B1). De préférence, la composition selon cette autre variante, comprend outre le monomère (A), un monomère (B) porteur de deux fonctions éther vinylique et/ou allylique, autre que le monomère (B1).

D'autres monomères (B) porteurs de deux fonctions éther vinylique et/ou allylique sont en effet déjà connus et/ou disponibles commercialement et peuvent être utilisés.

Il peut notamment s'agir d'un monomère (B2) comprenant deux fonctions éther vinylique et/ou allylique de formule générale:

**R₁-X_{B}-R₂**

dans laquelle
R₁ et R₂ sont identiques ou différents et sont choisis parmi le groupe allyle (-CH₂-CH=CH₂) et le groupe vinyle (-CH=CH₂) ;
Et X_{B} représente un atome, de préférence un atome d'oxygène, ou une chaîne linéaire acyclique longue d'au moins 2 atomes, de préférence choisie parmi :
   - une chaîne alkylène linéaire en C2 à C30, éventuellement substituée par un ou plusieurs groupes choisis parmi les groups alkyles, hydroxyalkyles, hydroxy, cétone, alkyloxy ;
   - une chaîne de type éther, notamment polyéther, par exemple polyéthylène glycol (PEG), polypropylène glycol (PPG), polytétraméthylène glycol (PTMG) et polyéther fluoré ;
   - les chaînes de type polysiloxane, notamment polydiméthylsiloxane (PDMS) ;
   - les chaînes de type polydiène, notamment polyisoprène et polybutadiène ; et
   - les chaînes de type polyester aliphatique ou semi-aliphatique, de faible température de transition vitreuse, comme par exemple la poly ε-caprolactone.

Par faible température de transition vitreuse, on entend généralement une température de transition vitreuse inférieure à 20°C, avantageusement inférieure à -20°C et préférentiellement inférieure à -35°C.

Le monomère (B2) est, de préférence, un monomère pour lequel X_{B} est une chaîne polyéther ou polyester aliphatique.

Le monomère (B2) peut présenter une masse molaire allant de 200 à 8000 g.mol⁻¹, préférentiellement de 300 à 4000 g.mol⁻¹.

L'introduction de ce type de monomère dans la composition selon l'invention est avantageuse car elle permet également de réduire le nombre de nœuds de réticulation de chaîne et assouplir le réseau obtenu après polymérisation de la composition.

La composition selon l'invention peut renfermer, outre le monomère (A), un mélange des monomères (B1) et (B2) décrits précédemment.

La composition selon l'invention peut en outre comprendre un monomère (C), différent des monomères (A) et (B), copolymérisable avec ces derniers.

Le monomère (C) est un monomère comprenant au moins deux fonctions maléate et/ou fumarate.

De préférence, la formule générale de ce monomère ou composé (C) est la suivante :

**R₃-X_{C}-R₄**

dans laquelle
R₃ et R₄ sont identiques ou différents et sont choisis parmi le groupe maléate (-O-C(O)-CH=CH-C(O)-OR en configuration cis) et le groupe fumarate (-O-C(O)-CH=CH-C(O)-OR en configuration trans), R représentant de façon indépendante un groupe alkyle comprenant de 1 à 10 atomes de carbone et l'atome d'hydrogène;
X_{C} représente une chaîne linéaire acyclique longue d'au moins 2 atomes, de préférence choisie parmi :
   - une chaîne alkylène linéaire en C2 à C30, éventuellement substituée par un ou plusieurs groupes choisis parmi les groupes alkyles, hydroxyalkyles, hydroxy, cétone, alkyloxy ;
   - une chaîne de type éther, notamment polyéthylène glycol (PEG), polypropylène glycol (PPG), polytétraméthylène glycol (PTMG) et polyéther fluoré ;
   - les chaînes de type polysiloxane, notamment polydiméthylsiloxane (PDMS) ;
   - les chaînes de type polydiène, notamment polyisoprène et polybutadiène ; et
   - les chaînes de type polyester aliphatique ou semi-aliphatique de faible température de transition vitreuse, comme par exemple la poly ε-caprolactone.

L'introduction de ce type de monomère (C) dans la composition selon l'invention est avantageuse car elle permet de moduler la distance entre les nœuds de réticulation de la chaîne et ainsi d'adapter les propriétés du réseau réticulé aux besoins.

Le monomère (C) est de préférence un dérivé maléique ou fumarique de polyéther (PEG/ PPG ou PTMG) ou de polyester aliphatique (poly ε-caprolactone par exemple).

Le monomère (C) peut présenter une masse allant de 200 à 8000 g.mol⁻¹, préférentiellement de 300 à 4000 g.mol⁻¹.

Dans la composition selon l'invention, le monomère (C) peut représenter de 0 à 50 %, préférentiellement de 0 à 30 %, plus préférentiellement de 1 à 25 % et encore plus préférentiellement de 2 à 20 % en moles de la composition.

Préférentiellement, le ratio molaire de (A+C)/(B) est compris entre 30/70 et 70/30, plus préférentiellement encore entre 55/45 à 45/55.

Le nombre total de moles de (A) peut être supérieur à 20% en moles, préférentiellement supérieur à 50% en moles, plus préférentiellement encore supérieur à 80% en moles de la somme de (A) et (C). Plus cette proportion est grande, plus la composition obtenue est riche en matière biosourcée, ce qui présente un avantage par rapport aux compositions actuellement existantes à base de dérivés pétroliers.

La composition selon l'invention peut en outre comprendre un autre monomère (D), différent des monomères (A) et (B) et de l'éventuel monomère (C), copolymérisable avec ces derniers.

Le monomère(D) est un monomère susceptible de réagir avec les monomères (A) et (B) et de l'éventuel monomère (C) comprenant au moins une fonction promotrice d'adhésion.

Une fonction promotrice d'adhésion est une fonction chimique susceptible d'établir des liaisons chimiques ou physiques fortes ou faibles avec la surface d'un substrat.

De préférence, la fonction promotrice d'adhésion peut être choisie parmi la fonction acide carboxylique, notamment les fonctions acrylique ou méthacrylique, les fonctions comprenant au moins un atome de phosphore sous forme acide ou ester, les fonctions éthoxy- ou méthoxy-silane ou les fonctions silanol.

Ce monomère (D) peut être l'acide acrylique, l'acide méthacrylique, les composés insaturés dérivés d'acide phosphorique et d'ester d'acide phosphorique, les composés insaturés alcoxysilanes, les composés insaturés porteurs de la fonction silanol.

Selon une variante, le monomère (D) comprend éventuellement en outre une fonction choisie parmi la fonction maléate, la fonction fumarate, la fonction éther vinylique et la fonction éther allylique.

Le monomère (D) peut représenter de 0 à 20%, préférentiellement de 1 à 15 %, plus préférentiellement de 1,5 à 10 % et encore plus préférentiellement de 1,7 à 5% en poids de la composition selon l'invention.

La composition selon l'invention peut également comprendre un autre monomère (E), différent des monomères (A) et (B), et des éventuels monomères (C) et/ou (D), copolymérisable avec ces derniers.

Le monomère (E) peut être notamment un monomère comprenant une seule fonction choisie parmi la fonction maléate, la fonction fumarate, la fonction éther vinylique et la fonction éther allylique, employé comme limitateur de chaînes, ce qui permet de moduler les propriétés du polymère obtenu, notamment sa résistance à l'abrasion et au choc. Cette modification des propriétés est permise car le limitateur de chaîne permet de diminuer la densité de nœuds de réticulation du polymère obtenu.

Le monomère (E) peut représenter de 0 à 50% %, préférentiellement de 1 à 25 %, plus préférentiellement de 2 à 15 % et encore plus préférentiellement de 2,5 à 5% en poids de la composition selon l'invention.

La composition selon l'invention peut comprendre, en outre, un amorceur de polymérisation.

Dans la présente invention, le terme « amorceur de polymérisation » désigne tout agent chimique capable de déclencher la réaction de polymérisation des monomères présents dans la composition.

Préférentiellement, l'amorceur est un amorceur radicalaire. Cet amorceur peut être un photoamorceur ou un amorceur thermique. L'amorceur est, de préférence, un photoamorceur.

Les amorceurs thermiques préférés pour la présente invention peuvent être choisis dans le groupe constitué par les peroxydes organiques, tels que le benzoyl peroxyde, le méthyl-cyclo-hexanone peroxyde, le cumènehydroperoxyde, le diisopropylbenzène peroxyde, le di-*t*-butyl peroxyde, le peroxybenzoate de *t*-butyle, le peroxycarbonate de diisopropyle et le monocarbonate de *t-*butylperoxyisopropyle, et par les composés azoïque, tels que le 2,2'-azobis-isobutyronitrile (AIBN).

Les photoamorceurs préférés pour la présente invention peuvent être choisis dans le groupe constitué par les cétones aromatiques, tels que la benzophénone, les composés aromatiques, tels que l'anthracène, l'α-chlorométhylnaphtalène et le diphénylsulfide, et les composés soufrés, tels que le thiocarbamate. En particulier, le photoamorceur peut être choisi parmi les photoamorceurs activés par irradiation UV, par exemple ceux du groupe constitué par :l'acétophénone, l'acétophénone benzylcétal, la 1-hydroxycyclohexyl phénylcétone, la 2,2-diméthoxy-1,2-diphényléthane-1-one, la xanthone, la fluorènone, le benzaldéhyde fluorène, l'anthraquinone, la triphénylamine, le carbazole, la3-méthyl-acétophénone, la 4-chloro-benzophénone, la 4,4'-méthoxy-benzophénone, la 4,4'-diamino-benzophénone, le benzoinpropyléther, le benzoinéthyl éther, la benzyl méthyl cétal 1-(4-isopropylphényl)-2-hydroxy-2-méthyl-propane-1-one, la 2-hydroxy-2-méthyl-1-phénylpropan-1-one, la thioxanthone, la diéthyl-thioxanthone, la 2-isopropyl-thioxanthone, la 2-chlorothioxanthone, la 2-méthyl-1-[4-(méthyl-thio)-phényl]-2-morpholinopropan-1-one, la 2-benzyl-2-diméthyl-amino-1-(4-morpholino-phényl)-butan-1,4-(2-hydroxyéthoxy)-phényl-(2-hydroxy-2-propyl) cétone, l'oxyde de 2,4,6-triméthylbenzoyl diphényl-phosphine, l'oxyde de bis-(2,6-méthoxybenzoyl)-2,4,4-triméthyl-pentyl phosphine, etl'oligo-(2-hydroxy-2-méthyl-1-(4-(1-méthylphényl)phényl)-propanone) .

Des photoamorceurs de polymérisation disponibles dans le commerce sont, par exemple, les produits Irgacure® 184, 369, 651, 500, 819, 907, 784, 2959, CGI 1700, CGI 1750, CGI 1850 et CG24_61, Darocur® 1116 et 1173, et Lucirin® TPO de BASF, le produit Uvecryl P36 d'UCB, et les produits Esacure KIP 150, KIP 65LT, KIP 100F, KT37, KT55, KTO46 et KIP75/B de Fratelli Lamberti.

L'amorceur de polymérisation peut consister en un mélange de plusieurs amorceurs de polymérisation, par exemple en un mélange d'un amorceur thermique et d'un photoamorceur.

La quantité massique d'amorceur pourra être adaptée par l'homme du métier. Cette quantité massique peut aller de 0,01 à 20 %, préférentiellement de 1 à 15 % de la masse totale des monomères (A), (B), (C), (D) et (E).

La composition peut également comprendre au moins un agent d'étalement ayant pour fonction de permettre, avant réticulation, un bon étalement de la composition lorsqu'elle est appliquée sur le substrat. L'agent d'étalement peut être particulièrement utile pour la formation de revêtements, en particulier lorsque le monomère (B) et/ou (C) est un monomère de type oligomérique, c'est-à-dire comprenant plusieurs motifs répétitifs, car ces monomères oligomériques peuvent rendre plus difficile l'application de la composition selon l'invention sur le substrat.

La composition peut également comprendre d'autres constituants non copolymérisables avec les monomères de la composition. Il peut s'agir par exemple d'inhibiteurs de polymérisation, de colorants ou de pigments, d'opacifiants ou d'additifs de protection thermique ou ultra-violet, des agents antibactérien, anti-salissure ou anti-fongiques. De préférence cependant, la composition selon l'invention comprend moins de 5 %, plus préférentiellement moins de 2% en poids de ces autres constituants.

Un avantage de l'invention est que la composition peut être fabriquée, à partir des monomères (A), (B), et des éventuels monomères (C), (D) et/ou (E), juste avant l'étape de réticulation. Ainsi les monomères conservés séparément gardent une excellente stabilité temporelle. La composition selon l'invention peut être obtenue par simple mélange des monomères (A) et (B), ainsi que des éventuels monomères (C), (D) et/ou (E), et d'éventuels autres constituants non copolymérisables.

Comme indiqué précédemment, la composition selon l'invention peut comprendre des inhibiteurs de polymérisation. En l'absence d'amorceur dans la composition, l'inhibiteur n'est pas nécessaire pour pouvoir conserver la composition. En revanche lorsque la composition renferme l'amorceur, on préféra ajouter à la composition un tel inhibiteur.

Cette composition se présente avantageusement sous forme liquide à température ambiante. La viscosité de la composition peut aller jusqu'à 15000 Poises à 25°C.

La composition selon l'invention, en particulier lorsqu'elle ne renferme pas d'amorceur, peut être conservée pendant plusieurs mois en l'absence de lumière à une température maximale de 25°C sans présenter de signes de dégradations ou de réticulation.

La composition peut être utilisée pour la fabrication de polymères réticulés. Ces polymères réticulés peuvent être formulés pour des applications de tout type. Il peut s'agir par exemple de résines composites comprenant la composition selon l'invention et des renforts (charge ou fibres).

En particulier, cette composition peut avantageusement être utilisée pour la fabrication de revêtements sur des substrats. Par « revêtement », on entend dans la présente invention tout type de couche de faible épaisseur appliquée sur un substrat, quelle que soit la fonction de cette couche. Il peut s'agir par exemple de revêtements protecteurs, décoratifs, ou de traitement de surface. Les films, les vernis et les peintures font partis des revêtements au sens de la présente invention.

Le substrat sur lequel est appliqué le revêtement dans la présente invention peut être de toute sorte. Il peut s'agir un particulier de substrats en bois, en métal, en plastique, en verre ou en papier.

L'invention a également pour objet un procédé de fabrication d'un revêtement polymère sur un substrat comprenant les étapes suivantes :
- le dépôt sur le substrat d'une couche de la composition selon l'invention telle que décrite ci-avant, puis
- la réticulation de la composition ainsi déposée.

Le dépôt de la composition peut être réalisé selon tout moyen connu de l'homme du métier, par exemple par trempage, par enduction centrifuge, par« hand coater », par « tape casting » ou par pulvérisation. L'épaisseur de la couche déposée est ajustée en fonction de l'épaisseur du revêtement que l'on souhaite obtenir. L'épaisseur de la couche déposée peut par exemples être comprise entre 100 nm et 2 mm. De préférence, la couche a une épaisseur uniforme, de manière à obtenir un revêtement final uniforme.

Le procédé d'activation de l'amorceur de polymérisation varie en fonction du type d'amorceur contenu dans la composition. La durée et les conditions de l'étape d'activation de l'amorceur de polymérisation dépendent essentiellement de la nature et de la concentration des monomères et de l'amorceur dans la composition, et l'homme du métier saura adapter ces paramètres selon le cas.

Selon un mode de réalisation préféré, l'amorceur de polymérisation est un photoamorceur, et l'activation de ce photoamorceur est obtenue en exposant la couche de composition déposée sur le substrat à une irradiation, en particulier à une irradiation UV. De préférence, l'irradiation est effectuée de façon égale sur l'ensemble de la couche de composition déposée sur le substrat.

La réticulation peut être effectuée par l'activation de l'amorceur de polymérisation. Cette activation permet de déclencher la réaction de polymérisation des monomères (A) et (B) entre eux, ainsi qu'avec d'autres monomères réactifs éventuellement présents, en particuliers les monomères (C), (D) et/ou (E) tels que décrits précédemment. Les monomères (A) et (B) polymérisent selon un système de type accepteur-donneur.

L'amorceur peut être déjà présent dans la composition ou ajouté juste avant le dépôt de la composition sur la surface à revêtir.

L'amorceur est de préférence ajouté juste avant le dépôt de la composition sur la surface à revêtir.

Des étapes additionnelles sont possibles comme un recuit du revêtement ou un flammage du support avant application de la composition, mais ces étapes ne sont pas obligatoires pour réaliser le procédé selon l'invention.

Ce procédé permet d'obtenir un revêtement polymère présentant des propriétés intéressantes. En particulier, les revêtements obtenus peuvent présenter au moins une des propriétés suivantes :
- une bonne transparence ;
- un indice de réfraction faible ;
- une dureté élevée ;
- une bonne adhésion au substrat ;
- une bonne résistance à l'abrasion ;
- une bonne résistance chimique, aux solvants par exemple à l'eau, mais aussi aux bases et aux acides ;
- une brillance élevée ;
- une bonne tenue à l'impact.

Ainsi, les revêtements obtenus qui sont également des objets de la présente invention possèdent des propriétés aussi bonnes voire meilleure que les revêtements actuellement disponibles, et ils présentent en outre l'avantage d'être obtenus aisément à partir de matériaux biosourcés.

L'invention sera mieux comprise à la lumière des exemples non limitatifs et purement illustratifs suivants.

### EXEMPLES

### Synthèse du monomère (A)

### Synthèse de l'hémi-ester maléique d'éthyle

On a introduit dans un réacteur d'une capacité de 250 mL, chauffé par un bain d'huile de silicone, muni d'un système d'agitation à barreau aimanté, 5,45 g d'anhydride maléique (0,56 mol), dissous dans de l'éthanol absolu (350 mL). On a agité le mélange pendant 24 heures à température ambiante. On a suivi l'avancement de la réaction par RMN ¹H (CDCl₃). On a évaporé l'éthanol sous pression réduite. On a obtenu 8 g de produit sous forme d'un liquide incolore (rendement 99%).

### Synthèse du di(maléate d'éthyle) isosorbide

On a introduit dans un réacteur d'une capacité de 250 mL, chauffé par un bain d'huile de silicone, muni d'un système de contrôle de la température du bain, d'un système d'agitation à barreau aimanté et d'un appareil de Dean Stark surmonté d'un réfrigérant, 1,7 g d'isosorbide (0,012 mol) dissous dans 100 mL de toluène, puis 5 g d'hémi-ester maléique d'éthyle (3 équivalents molaires par rapport à l'isosorbide) et 1 g d'acide para-toluènesulfonique. On a chauffé le mélange à reflux total pendant 24 heures. On a suivi l'avancement de la réaction par RMN ¹H (CDCl₃). On a évaporé le toluène sous pression réduite, puis on a lavé le brut trois fois avec une solution d'hydrogénocarbonate de sodium saturée et une fois avec une solution d'eau salée saturée. On a séché la phase organique avec du sulfate de magnésium anhydre et on a évaporé le solvant sous pression réduite. On a obtenu 4,3 g de produit sous forme d'huile jaunâtre (mélange contenant du monomère (majoritaire) et des oligomères) (rendement 93%).

### Monomère (B)

- divinyl éther d'isosorbide **(B1)**, obtenu à partir d'isosorbide selon le protocole décrit dans Zhurnal Obshchei Khimii, 1972, 42(10), p 2302-2303 par addition d'acétylène.
- diallyl éther d'isosorbide **(B1)**, obtenu à partir d'isosorbide selon le protocole décrit dans Zhurnal Obshchei Khimii, 1972, 42(10), p 2302-2303 par synthèse de type Williamson avec le bromure d'allyle.
- divinyle éther de 1,4-butane diol (**B2**) (Aldrich)
- diallyle éther (**B2**) (Aldrich)

**Amorceur** : Darocur® 1173(BASF)

### 1. Préparation de compositions réticulables

### a. Compositions selon l'invention :

Quatre compositions selon l'invention ont été préparées en mélangeant les composés dans les proportions indiquées dans le tableau suivant, les proportions étant indiquées en grammes.

| | **C.1** | **C.2** | **C.3** | **C.4** |
|---|---|---|---|---|
| **Monomère (A)** : | | | | |
| - di(maléate d'éthyle) isosorbide | 17,6 | 39,8 | 20,1 | 28,0 g |
| **Monomère** **(B1)** : | | | | |
| - divinyle éther d'isosorbide | | | 10,0 | |
| - diallyle éther d'isosorbide | 10,0 | | | |
| **Monomère (B2)** : | | | | |
| - divinyle éther de 1,4-butane diol | | | | 10,0 |
| - diallyle éther | | 10,0 | | |
| **Amorceur** Darocur® 1173 | 0,7 | 0,6 | 0,3 | 0,5 |

Les mélanges ont ensuite été homogénéisés par agitation et bain ultra-son.

### b. Composition non-conforme à l'invention, à titre comparatif :

Une composition (**CP 1**) a été préparée conformément à l'enseignement de l'exemple 9 de la demande WO 2011/048739. 10 g de diméthacrylate d'isosorbide ont été mélangés à 0.1 g d'amorceur (Darocur® 1173).

### 2. Test de conservation des monomères et des compositions renfermant ces monomères

Le di(maléate d'éthyle) isosorbide (DMEI), le divinyl éther d'isosorbide (DVEI), le diallyl éther isosorbide (DAEI), les compositions selon l'invention mentionnées ci-dessus, hormis qu'elles ne comprennent pas d'amorceur ainsi que la composition à base de diméthacrylate d'isosorbide telle que décrite ci-dessus, hormis là aussi qu'elle ne comprend pas d'amorceur, ont été soumis au test de stockage suivant :
Des échantillons sont placés dans les mêmes conditions pendant 15 jours dans un dessiccateur en atmosphère anhydre. Au bout de ces 15 jours, le taux de conversion (TC) de l'échantillon, exprimé en pourcentage (%), est déterminé par spectroscopie FT-IR.

Plus ce taux est faible, meilleure est la capacité au stockage de l'échantillon.

Les résultats suivants ont été obtenus:

| **Echantillon** | **DMEI** | **DVEI** | **DAEI** | **C1** | **C2** | **C3** | **C4** | **CP1** |
|---|---|---|---|---|---|---|---|---|
| **TC (%)** | 0% | 0% | 0% | 1% | 2% | 1% | 1% | 10% |

On voit que la stabilité au stockage des compositions selon l'invention est bien supérieure à celle de la composition décrite dans la demande WO 2011/048739.

Or, si la composition a déjà partiellement réagi, cela peut poser des problèmes de mise en forme du polymère réticulé, et particulièrement lorsque l'on souhaite former un revêtement.

Par ailleurs, lorsque les produits de la composition sont stockés séparément, ils sont parfaitement stables (aucune conversion observée). Ainsi, on peut aisément fabriquer la composition comprenant les différents monomères ainsi que l'amorceur juste avant d'effectuer la réticulation.

### 3. Réalisation des revêtements sur un support acier

Les supports suivants ont été utilisés : Lame Q-Lab de type Q-panel acier laminé 76x152 mm.

Une fine couche de composition réticulable telle que décrite ci-dessus (partie 2) a été déposée afin de recouvrir toute la surface du support avec le minimum de composition.

Les supports ainsi recouverts ont ensuite été déposés sur le tapis roulant d'un banc UV(Fusion UV System, Inc®, modèle LC6E - Lampe à vapeur de mercure de type H (FUSION F300S)- 200nm-600nm - 120 W.cm⁻² - quelques secondes d'expositions) afin de procéder à la polymérisation ou réticulation.

Pour chacune des compositions, 5 passages à 15 cm/s sous la lampe UV ont été réalisés. Des revêtements d'une épaisseur de 150 µm ont ainsi été obtenus.

### 4. Caractérisation/évaluation des propriétés des revêtements ainsi obtenus

**Le taux de conversion (TC)** exprimé en pourcentage (%) est déterminé par spectroscopie FT-IR, après 2 minutes d'irradiation UV (240-450nm) à 350 mW.cm⁻².

**La température de transition vitreuse(Tg)** exprimée en degré Celsius (°C) a été mesurée par calorimétrie différentielle à balayage (2 passages de -100°C à 200°C, 15°C.min⁻¹).

**La température de dégradation à 10% de perte de masse (Td₁₀)** exprimée en dégrée Celsius (°C) a été mesurée par analyse thermogravimétrique (25°C à 600°C, 10°C.min⁻¹ sous air).

Les **taux de gonflement** (en pourcentage %) dans le tétrahydrofurane **(TG_{THF})** (TG = (m_{abs}-mᵢ) / mᵢ).

Le **taux d'insolubilité** (en pourcentage %) a été calculé dans le dichlorométhane **(TI_{DCM})** (TI = m_{sec} /mᵢ).

| **Composition** | **TC (%)** | **(Tg °C)** | **Td₁₀ (°C)** | **TG (%) (THF)** | **TI (%) (DCM)** |
|---|---|---|---|---|---|
| **C.1** | 58 | NM | 333 | NM | NM |
| **C.2** | 40 | 36 | 268 | NM | NM |
| **C.3** | 67 | NM | 320 | NM | NM |
| **C.4** | 82 | NM | 296 | NM | NM |
| **CP 1** | 60 | 42 | 266 | 0 | 100± 0, 2 |

### (NM non mesuré)

Ces résultats montrent que l'on peut, à partir de compositions selon l'invention qui comprennent le monomère (A) (di(maléate d'alkyle) d'isosorbide), fabriquer des revêtements de polymères réticulés.

Ces revêtements présentent un aspect similaire au revêtement obtenu à partir de la composition à base de diméthacrylate d'isosorbide de l'art antérieur.

Les taux de conversion sont similaires voire supérieurs au taux de conversion obtenu pour le revêtement de la composition CP 1, ce qui montre la très bonne réactivité entre les monomères (A) et (B).
On peut également noter que l'on obtient que les polymères réticulés comprenant le di(maléate d'alkyle) d'isosorbide (A) présentent une stabilité thermique améliorée comme le montrent les Td₁₀ supérieures.

## Revendications

1. Composé (A) dérivé de 1,4:3,6-dianhydrohexitol porteur de deux fonctions maléate et/ou fumarate, choisi parmi les composés (A1) ou (A2) représentés ci-dessous ou un mélange de ces composés : 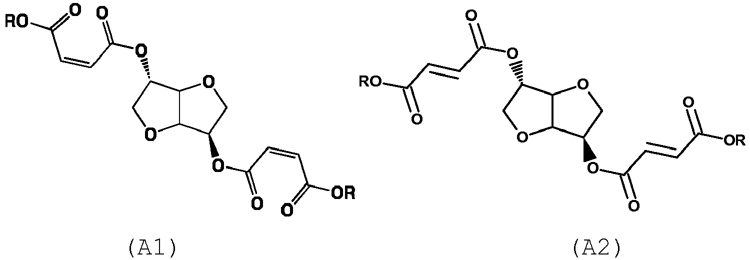 dans lesquelles R représente, de façon indépendante un groupe alkyle, de préférence un groupe alkyle comprenant de 1 à 10 atomes de carbone;

2. Procédé de préparation du composé (A) selon la revendication 1, comprenant une étape de réaction d'un hémi-ester d'acide butènedioïque avec du 1,4:3,6-dianhydrohexitol.

3. Polymère obtenu à partir du composé (A).

4. Composition comprenant :
- le composé (A) selon la revendication 1 et;
- un monomère (B) porteur de deux fonctions éther vinylique et/ou allylique.

5. Composition selon la revendication 4, dans laquelle le monomère (B) est un monomère de 1,4:3,6-dianhydrohexitol (B1).

6. Composition selon la revendication 5, dans laquelle le monomère (B1) est choisi parmi le divinyl éther d'isosorbide (B1a), le diallyl éther d'isosorbide (B1b) ou un mélange de divinyl éther d'isosorbide (B1a) et de diallyl éther d'isosorbide (B1b).

7. Composition selon la revendication 4, dans laquelle le monomère (B) est un monomère (B2) comprenant deux fonctions éther vinylique et/ou allylique de formule générale:
**R₁-X_{B}-R₂**
dans laquelle
R₁ et R₂ sont identiques ou différents et sont choisis parmi le groupe allyle et le groupe vinyle;
Et X_{B} représente un atome, de préférence un atome d'oxygène, ou une chaîne linéaire acyclique longue d'au moins 2 atomes, de préférence choisie parmi :
- une chaîne alkylène linéaire en C2 à C30, éventuellement substituée par un ou plusieurs groupes choisis parmi les groupes alkyles, hydroxyalkyles, hydroxy, cétone, alkyloxy ;
- une chaîne de type éther, notamment polyéther, par exemple polyéthylène glycol, polypropylène glycol, polytétraméthylène glycol et polyéther fluoré ;
- les chaînes de type polysiloxane, notamment polydiméthylsiloxane ;
- les chaînes de type polydiène, notamment polyisoprène et polybutadiène ; et
- les chaînes de type polyester aliphatique ou semi-aliphatique de faible température de transition vitreuse.

8. Composition selon la revendication 7, dans laquelle le monomère (B2) est un monomère pour lequel X_{B} est une chaîne polyéther ou polyester aliphatique.

9. Composition selon l'une quelconque des revendications 4 à 8, la composition comprenant en outre un monomère (C), différent des monomères (A) et (B), copolymérisable avec ces derniers, comprenant deux fonctions maléate et/ou fumarate.

10. Composition selon la revendication 9, dans laquelle le monomère (C) est choisi parmi les produits de formule suivante
**R₃-X_{C}-R₄**
dans laquelle
R₃ et R₄ sont identiques ou différents et sont choisis parmi le groupe maléate et le groupe fumarate, R représentant de façon indépendante un groupe alkyle comprenant de 1 à 10 atomes de carbone et l'atome d'hydrogène;
X_{C} représente une chaîne linéaire acyclique longue d'au moins 2 atomes, de préférence choisie parmi :
- une chaîne alkylène linéaire en C2 à C30, éventuellement substituée par un ou plusieurs groupes choisis parmi les groupes alkyles, hydroxyalkyles, hydroxy, cétone, alkyloxy ;
- une chaîne de type éther, notamment polyéthylène glycol, polypropylène glycol, polytétraméthylène glycol et polyéther fluoré ;
- les chaînes de type polysiloxane, notamment polydiméthylsiloxane ;
- les chaînes de type polydiène, notamment polyisoprène et polybutadiène ; et
- les chaînes de type polyester aliphatique ou semi-aliphatique de faible température de transition vitreuse, comme par exemple la poly ε-caprolactone.

11. Composition selon la revendication 10, dans laquelle le monomère (C) est un dérivé maléique ou fumarique de polyéther ou de polyester aliphatique.

12. Composition selon l'une quelconque des revendications 4 à 11, dans le ratio molaire de (A+C)/(B) est compris entre 30/70 et 70/30, plus préférentiellement encore entre 55/45 à 45/55.

13. Composition selon l'une quelconque des revendications 4 à 12, le nombre total de moles de (A) peut être supérieur à 20% en moles, préférentiellement supérieur à 50% en moles, plus préférentiellement encore supérieur à 80% en moles de la somme de (A) et (C).

14. Composition selon l'une quelconque des revendications 4 à 13, la composition comprenant en outre un monomère (D), différent des monomères (A) et (B) et de l'éventuel monomère (C), copolymérisable avec ces derniers, susceptible de réagir avec les monomères (A), (B) et (C) comprenant au moins une fonction promotrice d'adhésion.

15. Composition selon la revendication 14, le monomère D étant choisi parmi l'acide acrylique, l'acide méthacrylique, les composés insaturés dérivés d'acide phosphorique et d'ester d'acide phosphorique, les composés insaturés alcoxysilanes, et les composés insaturés porteurs de la fonction silanol.

16. Composition selon l'une quelconque des revendications 4 à 15, la composition comprenant en outre un autre monomère (E), différent des monomères (A), (B), (C) et (D), copolymérisable avec ces derniers et comprenant une seule fonction choisie parmi la fonction maléate, la fonction fumarate, la fonction éther vinylique et la fonction éther allylique.

17. Procédé de fabrication d'un revêtement polymère sur un substrat comprenant les étapes suivantes :
- le dépôt sur le substrat d'une couche de la composition selon l'une quelconque des revendications 4 à 16, puis
- la réticulation de la composition.

18. Revêtement susceptible d'être obtenu par le procédé tel que défini dans la revendication 17.

## Patentansprüche

1. Verbindung (A), abgeleitet von 1,4:3,6-Dianhydrohexitol, Träger zweier Maleat- und/oder Fumaratfunktionen, gewählt aus den unten dargestellten Verbindungen (A1) oder (A2) oder einer Mischung dieser Verbindungen: 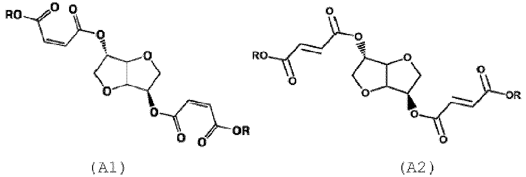 wobei R unabhängig für eine Alkylgruppe, bevorzugt eine Alkylgruppe mit 1 bis 10 Kohlenstoffatomen, steht.

2. Verfahren zur Herstellung der Verbindung (A) nach Anspruch 1, umfassend einen Reaktionsschritt eines Butendisäurehalbesters mit 1,4:3,6-Dianhydrohexitol.

3. Polymer, welches aus der Verbindung (A) erhalten wurde.

4. Zusammensetzung, umfassend:
- die Verbindung (A) nach Anspruch 1; und
- ein Monomer (B), das Träger zweier Vinyl- und/oder Allyletherfunktionen ist.

5. Zusammensetzung nach Anspruch 4, wobei das Monomer (B) ein 1,4:3,6-Dianhydrohexitol-Monomer (B1) ist.

6. Zusammensetzung nach Anspruch 5, wobei das Monomer (B1) gewählt ist aus Isosorbiddivinylether (B1a), Isosorbiddiallylether (B1b) oder einer Mischung aus Isosorbiddivinylether (B1a) und Isosorbiddiallylether (B1b).

7. Zusammensetzung nach Anspruch 4, wobei das Monomer (B) ein Monomer (B2) ist, das zwei Vinyl- und/oder Allyletherfunktionen der allgemeinen Formel:
**R₁-X_{B}-R₂**
umfasst,
wobei
R1 und R2 gleich oder verschieden sind und aus der Allylgruppe und der Vinylgruppe gewählt sind;
und wobei X_{B} für ein Atom steht, bevorzugt ein Sauerstoffatom, oder eine azyklische lineare Kette mit mindestens 2 Atomen, bevorzugt gewählt aus:
- einer linearen C2-C30-Alkylenkette, gegebenenfalls substituiert durch eine oder mehrere Gruppen, gewählt aus Alkyl-, Hydroxyalkyl-, Hydroxy-, Keton-, Alkyloxygruppen;
- eine Kette vom Ethertyp, insbesondere Polyether, beispielsweise Polyethylenglykol, Polypropylenglykol, Polytetramethylenglykol und fluorierter Polyether;
- Ketten vom Polysiloxantyp, insbesondere Polydimethylsiloxan;
- Ketten vom Polydientyp, insbesondere Polyisopren- und Polybutadien; und
- Ketten vom Typ aliphatische oder semi-aliphatische Polyester mit niedriger Glasübergangstemperatur.

8. Zusammensetzung nach Anspruch 7, wobei das Monomer (B2) ein Monomer ist, bei welchem X_{B} eine Polyether- oder aliphatische Polyesterkette ist.

9. Zusammensetzung nach einem der Ansprüche 4 bis 8, wobei die Zusammensetzung ferner ein Monomer (C) umfasst, das sich von den Monomeren (A) und (B) unterscheidet, mit diesen copolymerisierbar ist und zwei Maleat- und/oder Fumaratfunktionen umfasst.

10. Zusammensetzung nach Anspruch 9, wobei das Monomer (C) gewählt ist aus Produkten der folgenden Formel
**R₃-X_{c}-R₄**
wobei
R3 und R4 gleich oder verschieden sind und aus der Maleatgruppe und der Fumaratgruppe gewählt sind, R unabhängig für eine Alkylgruppe steht mit 1 bis 10 Kohlenstoffatomen und dem Wasserstoffatom;
Xc für eine azyklische lineare Kette mit wenigstens 2 Atomen steht, bevorzugt gewählt aus:
- einer linearen C2-C30-Alkylenkette, gegebenenfalls substituiert durch eine oder mehrere Gruppen, gewählt aus Alkyl-, Hydroxyalkyl-, Hydroxy-, Keton-, Alkyloxygruppen;
- einer Kette vom Ethertyp, insbesondere Polyethylenglykol, Polypropylenglykol, Polytetramethylenglykol und fluorierter Polyether;
- Ketten vom Polysiloxantyp, insbesondere Polydimethylsiloxan
- Ketten vom Polydientyp, insbesondere Polyisopren und Polybutadien; und
- Ketten vom Typ aliphatischer oder semialiphatischer Polyester mit niedriger Glasübergangstemperatur, wie beispielsweise Poly-ε-caprolacton.

11. Zusammensetzung nach Anspruch 10, wobei das Monomer (C) ein Maleinsäure- oder Fumarsäurederivat von Polyether oder aliphatischem Polyester ist.

12. Zusammensetzung nach einem der Ansprüche 4 bis 11, wobei das Molverhältnis (A+C)/(B) zwischen 30/70 und 70/30, noch stärker bevorzugt zwischen 55/45 und 45/55 liegt.

13. Zusammensetzung nach einem der Ansprüche 4 bis 12, wobei die Gesamtmolzahl (A) größer als 20 Mol-%, bevorzugt größer als 50 Mol-%, stärker bevorzugt größer als 80 Mol-% der Summe aus (A) und (C) sein kann.

14. Zusammensetzung nach einem der Ansprüche 4 bis 13, wobei die Zusammensetzung ferner ein Monomer (D) umfasst, das sich von den Monomeren (A) und (B) und von dem möglichen Monomer (C) unterscheidet, copolymerisierbar mit diesen und in der Lage, mit den Monomeren (A), (B) und (C) zu reagieren, die wenigstens eine haftungsfördernde Funktion umfassen.

15. Zusammensetzung nach Anspruch 14, wobei das Monomer D gewählt ist aus Acrylsäure, Methacrylsäure, ungesättigten Verbindungen, die von Phosphorsäure und Phosphorsäureester abgeleitet sind, ungesättigten Alkoxysilanverbindungen und ungesättigten Verbindungen, die Träger der Silanolfunktion sind.

16. Zusammensetzung nach einem der Ansprüche 4 bis 15, wobei die Zusammensetzung ferner ein anderes Monomer (E) umfasst, das sich von den Monomeren (A), (B), (C) und (D) unterscheidet, copolymerisierbar mit diesen und eine einzelne Funktion umfassend, die gewählt ist aus Maleatfunktion, Fumaratfunktion, Vinyletherfunktion und Allyletherfunktion.

17. Verfahren zur Herstellung einer Polymerbeschichtung auf einem Substrat, umfassend die folgenden Schritte:
- Aufbringen einer Schicht der Zusammensetzung nach einem der Ansprüche 4 bis 16 auf dem Substrat, dann
- Vernetzen der Zusammensetzung.

18. Beschichtung, die mittels des Verfahrens gemäß der Definition in Anspruch 17 erhalten werden kann.

## Claims

1. Compound (A) derived from 1,4:3,6-dianhydrohexitol carrying two maleate and/or fumarate functions, chosen from the compounds (A1) or (A2) shown below or a mixture of these compounds: 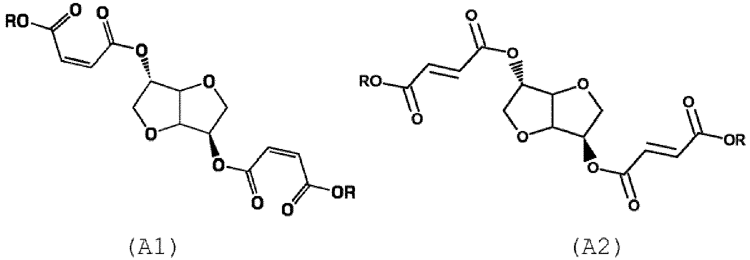 in which R represents, independently an alkyl group, preferably an alkyl group comprising from 1 to 10 atoms of carbon;

2. Method for preparing the compound (A) according to claim 1, comprising a step of reaction of a hemi-ester of butenedioic acid with 1,4:3,6-dianhydrohexitol.

3. Polymer obtained from the compound (A).

4. Composition comprising:
- the compound (A) according to claim 1 and;
- a monomer (B) carrying two vinyl ether and/or allyl functions.

5. Composition according to claim 4, wherein the monomer (B) is a monomer of 1,4:3,6-dianhydrohexitol (B1).

6. Composition according to claim 5, wherein the monomer (B1) is chosen from isosorbide divinyl ether (B1a), isosorbide diallyl ether (B1b) or a mixture of isosorbide divinyl ether (B1a) and isosorbide diallyl ether (B1b).

7. Composition according to claim 4, wherein the monomer (B) is a monomer (B2) comprising two vinyl and/or allyl ether functions having the general formula:
**R₁-X_{B}-R₂**
in which
R₁ and R₂ are identical or different and are chosen from the allyl group and the vinyl group;
And X_{B} represents an atom, preferably an atom of oxygen, or an acyclic linear chain at least 2 atoms long, preferably chosen from:
- a C2 to C30 linear alkylene chain, optionally substituted by one or more groups chosen from the alkyl, hydroxyalkyl, hydroxy, ketone, alkyloxy groups;
- a chain of the ether type, namely polyether, for example polyethylene glycol, polypropylene glycol, polytetramethylene glycol and fluorinated polyether;
- the chains of the polysiloxane type, namely polydimethylsiloxane;
- the chains of the polydiene type, namely polyisoprene and polybutadiene; and
- the chains of the aliphatic or semi-aliphatic polyester type having a low glass transition temperature.

8. Composition according to claim 7, wherein the monomer (B2) is a monomer for which X_{B} is a polyether or aliphatic polyester chain.

9. Composition according to any one of claims 4 to 8, the composition further comprising a monomer (C), different than the monomers (A) and (B), copolymerisable with the latter, comprising two maleate and/or fumarate functions.

10. Composition according to claim 9, wherein the monomer (C) is chosen from the products having the following formula
**R₃-X_{C}-R₄**
in which
R₃ and R₄ are identical or different and are chosen from the maleate group and the fumarate group, R independently representing an alkyl group comprising from 1 to 10 atoms of carbon and the atom of hydrogen;
Xc represents an acyclic linear chain at least 2 atoms long, preferably chosen from:
- a C2 to C30 linear alkylene chain, optionally substituted by one or more groups chosen from the alkyl, hydroxyalkyl, hydroxy, ketone, alkyloxy groups;
- a chain of the ether type, namely polyethylene glycol, polypropylene glycol, polytetramethylene glycol and fluorinated polyether;
- the chains of the polysiloxane type, namely polydimethylsiloxane;
- the chains of the polydiene type, namely polyisoprene and polybutadiene; and
- the chains of the aliphatic or semi-aliphatic polyester type having a low glass transition temperature, for example such as poly ε-caprolactone.

11. Composition according to claim 10, wherein the monomer (C) is a maleic or fumaric derivative of polyether or of aliphatic polyester.

12. Composition according to any one of claims 4 to 11, in the molar ratio of (A+C)/(B) is between 30/70 and 70/30, even more preferably between 55/45 to 45/55.

13. Composition according to any one of claims 4 to 12, the total number of moles of (A) can be greater than 20% in moles, preferably greater than 50% in moles, even more preferably greater than 80% in moles of the sum of (A) and (C).

14. Composition according to any one of claims 4 to 13, the composition further comprising a monomer (D), different than the monomers (A) and (B) and the possible monomer (C), copolymerisable with said monomers, capable of reacting with the monomers (A), (B) and (C) comprising at least one adhesion-promoter function.

15. Composition according to claim 14, the monomer (D) being chosen from acrylic acid, methacrylic acid, the unsaturated compounds derived from phosphoric acid and from phosphoric acid ester, the alkoxysilane unsaturated compounds, and the unsaturated compounds carrying the silanol function.

16. Composition according to any one of claims 4 to 15, the composition further comprising another monomer (E), different than the monomers (A), (B), (C) and (D), copolymerisable with the latter and comprising a single function chosen from the maleate function, the fumarate function, the vinyl ether function and the allyl ether function.

17. Method for manufacturing a polymer coating on a substrate comprising the following steps:
- the deposition on the substrate of a layer of the composition according to any one of claims 4 to 16, then
- the cross-linking of the composition.

18. Coating capable of being obtained by the method as defined in claim 17.
